# EUROPEAN PATENT APPLICATION

(11) **EP 4 297 040 A1**
(43) Date of publication of application: **27.12.2023**
(21) Application number: 22180426.3
(22) Date of filing: 22.06.2022
(51) Int. Cl.: G16H 20/13, G16H 30/40, G16H 40/63, G16H 40/67, A61J 1/00

(54) **METHOD FOR MONITORING THE TAKING OF A MEDICATION AND A MEDICATION SYSTEM**

(71) Applicant: Evondos Oy, 24240 Salo (FI)
(72) Inventor: Mäkelä, Markus, 21420 Lieto (FI); Apell, Mika, 20810 Turku (FI); Saarainen, Ville, 02650 Espoo (FI)
(74) Representative: Berggren Oy

(57) **Abstract**

The present invention provides a method for monitoring the taking of a medication. In the method a medication dispenser (201) is used to dispense the medication to a patient, a digital camera (216) is used to record a digital video of the patient, and the digital video is analysed to determine if the patient has taken the medication. The invention also relates to a medication system.

## Description

### TECHNICAL FIELD OF THE INVENTION

The present invention relates to a method for monitoring the taking of a medication and a medication system according to the preambles of the appended independent claims.

### BACKGROUND OF THE INVENTION

Patients should take their medications as prescribed to manage their health. Unfortunately, poor compliance to prescribed medications is a common problem that very often goes even unnoticed. Many patients simply forget to take their medications because no one reminded them to take or because they were confused by all the medications they were required to take. Medication compliance is especially difficult for elderly patients who are likely to have multiple conditions and take different medications with different dosing schedules. This can be especially difficult after being discharged from a hospital and on a new treatment schedule.

Medication compliance is paramount whether patients are alone at home in charge of their own medications, being seen by caregivers or living in a care facility. The confusion associated with a variety of medications can lead to unseen complications regardless of the setting. When patients understand their medications and the schedule they are following, that confidence leads to a sense of independence and increased compliance.

The simplest means of promoting medication compliance is through a pill organizer. The pill organizers range from a simple weekly unit divided into a plurality of compartments to more complicated monthly organizers that coordinate medication taking from compartments via integration with an app on a mobile phone. Because the pill organizer must be filled manually by the patient or the caregiver, there is a considerable risk that one or more of the compartments of the pill organizer are wrongly filled.

An automated medication dispenser solves the above problem of the pill organizers. The automated medication dispenser uses pre-packaged medications, which are dispensed to the patient as prescribed. The medications are typically pre-packaged into sachets that are connected to each other in chronological order to form a continuous strip.

Although the known pill organizers and automated medication dispensers improve medication compliance, there is still room for improvement. An existing problem with the pill organizers and automated medication dispensers is that it still cannot be known if the patient has actually taken the medications.

### OBJECTIVES OF THE INVENTION

It is the main objective of the present invention to reduce or even eliminate the prior art problems presented above.

It is an objective of the present invention to provide a method and a system that improve medication compliance. In more detail, it is an objective of the invention to provide a method and a system enabling to verify the taking of a medication. It is a further objective of the invention to provide a method and a system enabling to monitor the taking of a medication independently of time and place.

In order to realise the above-mentioned objectives, the method and the system according to the invention are characterised by what is presented in the characterising portions of the appended independent claims. Advantageous embodiments of the invention are described in the dependent claims.

### DESCRIPTION OF THE INVENTION

A method according to the invention for monitoring the taking of a medication comprises using a medication dispenser to dispense the medication to a patient, using a digital camera to record a digital video of the patient, and analysing the digital video to determine if the patient has taken the medication.

A medication system according to the invention comprises a medication dispenser configured to dispense a medication to a patient, a digital camera configured to record a digital video of the patient, and means for analysing the digital video to determine if the patient has taken the medication.

The method and the system according to the invention enable to verify the taking of a medication. By the taking of a medication is meant that the patient takes the medication orally, i.e., that the patient eats the medication. The taking of a medication may also contain the action of the patient taking the dispensed medication from the medication dispenser.

In the present invention, the medication dispenser is used to dispense the medication to the patient. The medication dispenser assists the patient in complying with his or her medical regimen by dispensing the correct medications at the correct times to the patient. The medications to be dispensed are preferably pre-packaged into sachets, which are connected to each other in chronological order so that they form a continuous strip. The medication dispenser dispenses the medications from the continuous strip one medication sachet at a time according to the medical regimen. The medications can be given to the patient with or without the sachet. The medication sachets can be provided with labels containing, for example, text, bar codes, and/or images that may include information about the patient such as his or her name, date of birth or social security number, and information about the medication such as name(s), dosage(s) and/or taking time.

The medication dispenser may comprise a container for a continuous strip of medication sachets, from which container the medication sachets are conveyed with conveying means to an outlet of the medication dispenser. The patient takes the medications from the outlet of the medication dispenser. The conveying means may comprise, for example, rollers and/or conveyors for conveying the continuous strip of medication sachets and/or medication sachets that have been detached from the continuous strip. The medication dispenser may comprise a cutter arranged in connection with the conveying means for detaching the medication sachets one by one from the continuous strip. The cutter can also be used to partly open the medication sachet, whereby the patient can easily take out the medication from the medication sachet.

The medication dispenser may comprise an internal digital camera that is arranged in connection with the conveying means for imaging a continuous strip of medication sachets. The internal digital camera enables to determine information from the labels of the medication sachets and/or to determine the locations of seams between the medication sachets. The internal digital camera may be configured to capture more than one image for the purpose of determining information from one label or determining the location of one seam. Between the capturing of images, the internal digital camera and/or the continuous strip can be moved and/or the lighting conditions can be changed. Various image processing techniques can be utilised for combining the images, for example, HDR (High Dynamic Range) and stereo imaging techniques.

The medication dispenser may comprise a control unit for controlling the operation of the medication dispenser. The control unit can be implemented in hardware, software, or a combination of hardware and software components. Hardware components may comprise a processor for processing data and a storage medium for storing the data. Software components may be in the form of computer-readable program code stored in a computer-readable storage medium such as memory, mass storage device, or removable storage device. For example, a computer-readable medium may comprise computer-readable code for performing the function of a particular component. Likewise, computer memory may be configured to include one or more components, which may then be executed by a processor. Components may be implemented separately in multiple modules or together in a single module. In particular, the control unit may comprise a processor that is programmed to carry out the functions that are needed to operate the medication dispenser, and a memory for storing, for example, the medical regimen of the patient. The medical regimen can be received from a server over a communications network.

The medication is dispensed to the patient at a prescribed time. The prescribed time is the taking time of the medication. The taking time can be obtained from the medical regimen of the patient. The medical regimen can be stored on the medication dispenser, or on a server that can be accessed over a communications network. In a case where the medications are pre-packaged into sachets, the taking times can alternatively be read from labels of the medication sachets. The medication dispenser can be configured to dispense the medication automatically at the taking time or the dispensation of the medication may require a certain action from the patient, such as using a button or a touch screen of the medication dispenser.

The dispensed medication may contain one or more tablets and/or capsules. The tablets are made by compressing one or more powdered active ingredients to form a hard, solid, smooth-coated pill that breaks down in the digestive tract. In addition to the active ingredients, the tablets typically contain additives that hold the pill together and improve the taste, texture, or appearance. The capsules include an outer shell that breaks down in the digestive tract. The outer shell can be filled with dry medication in powder or pellet form, medication in liquid form, or medication suspended in gelatine or a similar substance.

In the present invention, the digital camera is used to record the digital video of the patient. The digital camera may comprise a memory for storing the digital video, or the digital video can be stored into a memory of the medication dispenser. Preferably, the digital camera is connected to the control unit of the medication dispenser. In this case, the control unit can be configured to control the operation of the digital camera and the digital video can be stored into the memory of the control unit. The control unit and thus the digital camera can be controlled remotely over a communications network. The digital video can be transmitted from the medication dispenser to a server over a communications network. The digital video can be transmitted to the server directly from the digital camera or from the memory of the medication dispenser. The server may comprise a database for the recorded digital videos. The digital video can be linked with information about the patient such as his or her name, date of birth or social security number, and information about the medication such as name(s), dosage(s) and/or taking time.

The digital camera can be attached to or be integrated into the medication dispenser. The digital camera may comprise an adjustable base or support that enables an easy adjustment of the digital camera towards the patient. The digital camera may comprise a wide-angle lens. By a wide-angle lens is meant a lens whose focal length is substantially smaller than the focal length of a normal lens. The angle of view (AOV) of the wide-angle lens can be, for example, between 60° and 110°. The use of a wide-angle lens reduces or even eliminates the need for adjusting the orientation of the digital camera.

The recording of the digital video can be started at the time the medication is dispensed to the patient or at a predetermined time before or after the dispensation of the medication. Alternatively, the recording of the digital video can be started at the time the patient takes the medication from the medication dispenser or at a predetermined time after the patient has taken the medication from the medication dispenser. The medication dispenser may comprise a sensor for detecting the taking of the medication from the medication dispenser. Alternatively, the recording of the digital video can be started when the motion of the patient is detected. The digital camera or a motion detector can be used for detecting the motion of the patient. The recording of the digital video can be continued for a predetermined time or until the motion of the patient is not detected or has not been detected for a predetermined time. Alternatively, the recording of the digital video can be continued until the taking of the medication has been verified.

In the present invention, the digital video is analysed to determine if the patient has taken the medication. The digital video can be analysed with a neural network, a frame-by-frame video motion analysis or a digital signal processing analysis technique. The determination if the patient has taken the medication can be based on the movement of a body, the opening of a mouth, or the movement of swallowing. If the taking of the medication can be verified, the time when the patient has taken the medication can be determined and stored in a memory. If the taking of the medication cannot be verified, an alarm can be given to the patient and/or the caregiver. The digital video can be analysed at the medication dispenser or at a server to which the digital video has been transmitted over a communications network. The digital video can be analysed while the digital video is being recorded or after the digital video has been recorded. The digital video can be made available to the caregiver who may then watch the digital video to determine if the patient has taken the medication.

An advantage of the present invention is that it improves medication compliance. Another advantage of the invention is that it enables to verify the taking of a medication. Still another advantage of the invention is that it enables to monitor the taking of a medication independently of time and place.

According to an embodiment of the invention the digital video is transmitted to a server over a communications network, and the digital video is analysed at the server. The digital video can be stored on the medication dispenser, preferably on the memory of the control unit, from which the digital video is transmitted to the server. The medication dispenser comprises a communication interface for communicating with the server and thus enabling to transmit the digital video to the server. The server can be implemented in hardware, software, or a combination of hardware and software components. Hardware components may comprise a processor for processing data and a storage medium for storing the data. Software components may be in the form of computer-readable program code stored in a computer-readable storage medium such as memory, mass storage device, or removable storage device. For example, a computer-readable medium may comprise computer-readable code for performing the function of a particular component. Likewise, computer memory may be configured to include one or more components, which may then be executed by a processor. Components may be implemented separately in multiple modules or together in a single module. In particular, the server may comprise a processor, a memory, and a computer program stored in the memory that, when executed by the processor, analyses the digital video stored in the memory. An advantage of performing the analysis at the server is that the medication dispenser does not need to have a high computational capacity.

According to an embodiment of the invention the digital video is analysed at the medication dispenser. Preferably, the control unit of the medication dispenser is configured to analyse the digital video. The control unit may comprise a processor, a memory, and a computer program stored in the memory that, when executed by the processor, analyses the digital video stored in the memory. The result of the analysis can be transmitted to a server over a communications network. An advantage of performing the analysis at the medication dispenser is that it reduces the amount of data that is transferred between the medication dispenser and the server. Another advantage of performing the analysis at the medication dispenser is that it reduces transferring and processing of sensitive video data outside the medication dispenser.

According to an embodiment of the invention the step of using a digital camera to record a digital video of the patient comprises adjusting continuously or at predetermined time intervals the orientation of the digital camera so that the digital camera is directed towards the patient's body. The digital camera can be configured to determine the patient's location based on which the orientation of the digital camera can be adjusted. Preferably, the digital camera is directed towards the patient's head. The time interval between the adjustments can be, for example, 1-2 seconds, 2-5 seconds, or 5-20 seconds. An advantage of adjusting the orientation of the digital camera is that the digital camera can be kept directed towards the patient despite of his or her movement.

According to an embodiment of the invention the digital video is recorded with a plurality of digital cameras arranged to capture the patient from different angles. The digital cameras are preferably evenly distributed around the room where the medication dispenser is located. The number of digital cameras can be, for example, 2-5, 5-10, or 10-20. An advantage of using a plurality of digital cameras to capture the patient from different angles is that it facilitates the verifying of the taking of the medication.

According to an embodiment of the invention the recording of the digital video is started at the time the medication is dispensed to the patient. The medication dispenser can be configured to dispense the medication automatically at the taking time of the medication, or the dispensation of the medication may require a certain action from the patient, such as using a button or a touch screen of the medication dispenser.

According to an embodiment of the invention the recording of the digital video is started at the time the patient takes the medication from the medication dispenser. The medication dispenser may comprise an optical or mechanical sensor for detecting the taking of the medication from the medication dispenser.

According to an embodiment of the invention the recording of the digital video is started when the motion of the patient is detected. In other words, the recording of the digital video is triggered by the motion of the patient. The digital camera or a motion detector can be used for detecting the motion of the patient.

According to an embodiment of the invention the recording of the digital video is continued until the motion of the patient is not detected or has not been detected for a predetermined time. The predetermined time can be, for example, 1-10 seconds, 10-30 seconds, or 30-60 seconds. The digital camera or a motion detector can be used for detecting the motion of the patient.

According to an embodiment of the invention the recording of the digital video is continued until the taking of the medication has been verified. In this embodiment, the digital video is analysed while the digital video is being recorded.

According to an embodiment of the invention the determination if the patient has taken the medication is based on at least one of the following: movement of a body, opening of a mouth, and movement of swallowing. If the determination is based on the movement of a body, then preferably it is based on the movement of a hand.

According to an embodiment of the invention the digital video is analysed using one of the following techniques: a neural network, a frame-by-frame video motion analysis or a digital signal processing analysis. If the result of the analysis is uncertain, the caregiver may watch the digital video to determine if the patient has taken the medication.

According to an embodiment of the invention the method comprises, if the taking of the medication has been verified, determining the time when the patient has taken the medication.

According to an embodiment of the invention the method comprises, if the taking of the medication has not been verified, giving an alarm to the patient and/or the caregiver. After the caregiver has been notified, the caregiver may re-evaluate the situation by watching the digital video to determine if the patient has taken the medication.

According to an embodiment of the invention the method comprises, after the medication has been dispensed from the medication dispenser to the patient, instructing the patient to take the medication. The patient can be instructed with audio and video instructions by using, for example, a loudspeaker and a display of the medication dispenser.

According to an embodiment of the invention the medication system comprises means for transmitting the digital video to a server over a communications network, and the means for analysing the digital video to determine if the patient has taken the medication are located at the server.

According to an embodiment of the invention the means for analysing the digital video to determine if the patient has taken the medication are located at the medication dispenser.

According to an embodiment of the invention the medication system comprises means for adjusting continuously or at predetermined time intervals the orientation of the digital camera so that the digital camera is directed towards the patient's head.

According to an embodiment of the invention the medication system comprises a plurality of digital cameras arranged to capture the patient from different angles.

According to an embodiment of the invention the medication system comprises a motion detector configured to detect the motion of the patient.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

The exemplary embodiments presented in this text and their advantages relate by applicable parts to the method as well as the system according to the invention, even though this is not always separately mentioned.

### BRIEF DESCRIPTION OF THE DRAWINGS

- Fig. 1: illustrates a flow chart of a method according to an embodiment of the invention, and
- fig. 2: illustrates a system according to an embodiment of the invention.

### DETAILED DESCRIPTION OF THE DRAWINGS

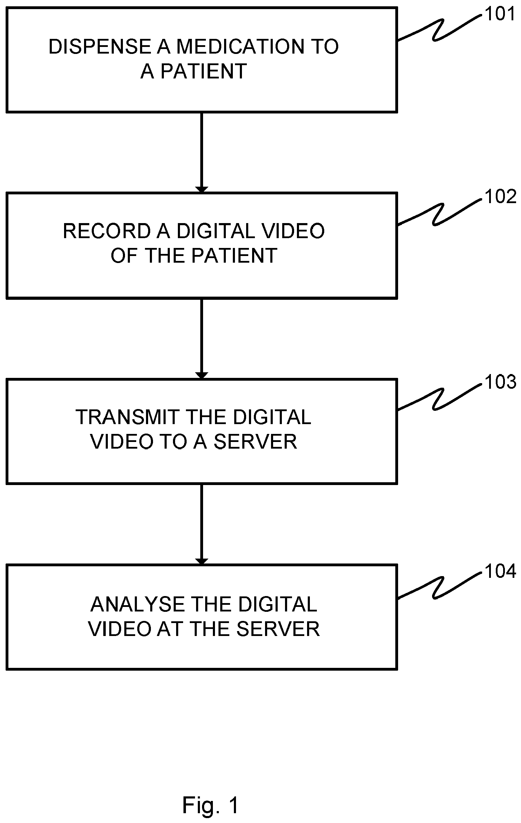

Fig. 1 illustrates a flow chart of a method according to an embodiment of the invention. The method enables to monitor and verify the taking of a medication.

At step 101, a medication is dispensed to a patient by using a medication dispenser. The medication is dispensed to the patient at a prescribed time specified in the medical regimen of the patient.

At step 102, a digital video of the patient is recorded by using a digital camera. The recording of the digital video is started at the time the patient takes the medication from the medication dispenser and is continued for a predetermined time. The medication dispenser comprises a sensor for detecting the taking of the medication from the medication dispenser. The digital video is stored into a memory of the medication dispenser and embedded with information about the patient such as his or her name, date of birth or social security number, and information about the medication such as name(s), dosage(s) and/or taking time.

At step 103, the digital video is transmitted to a server. The digital video is transmitted to the server from the memory of the medication dispenser over a communications network. At the server, the digital video is stored into a database.

At step 104, the digital video is analysed at the server to determine if the patient has taken the medication. The digital video is analysed with a neural network, a frame-by-frame video motion analysis or a digital signal processing analysis technique. The determination if the patient has taken the medication is based on the movement of a body, the opening of a mouth, or the movement of swallowing.

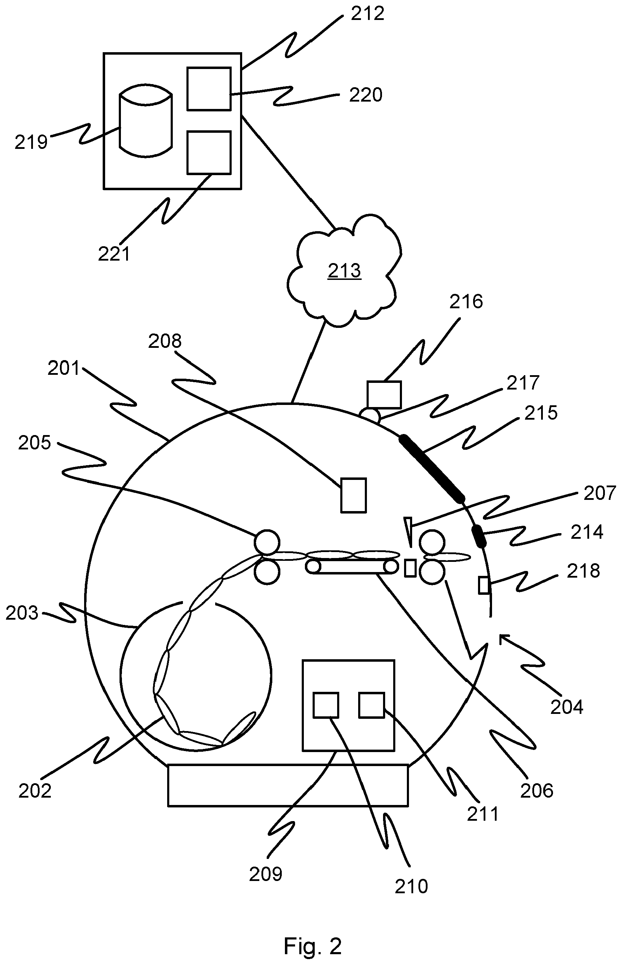

Fig. 2 illustrates a system according to an embodiment of the invention. The system comprises a medication dispenser 201 that dispenses the correct medications at the correct times to a patient. The medication dispenser 201 is shown as a cross sectional view in fig. 2. The medications to be dispensed are pre-packaged into sachets 202, which are connected to each other in chronological order so that they form a continuous strip. The medication sachets 202 are provided with labels (not shown in fig. 2) containing text, bar codes, and/or images that include information about the patient such as his or her name, date of birth or social security number, and information about the medication such as name(s), dosage(s) and/or taking time.

The medication dispenser 201 comprises a container 203 for the continuous strip of medication sachets 202, from which container 203 the medication sachets 202 are conveyed with conveying means to an outlet 204. The conveying means comprise rollers 205 and a conveyor 206 for conveying the continuous strip of medication sachets 202 or separated medication sachets 202. The medication dispenser 201 comprises a cutter 207 arranged in connection with the conveying means for detaching the medication sachets 202 one by one from the continuous strip. The cutter 207 is also used to partly open the medication sachet 202 to be dispensed, whereby the patient can easily take out the medication from the medication sachet 202. The medication dispenser 201 comprises a digital camera 208 that is arranged in connection with the conveying means for imaging the continuous strip. The digital camera 208 enables to determine information from the labels of the medication sachets 202 and to determine the locations of seams between the medication sachets 202. The medication dispenser 201 comprises a control unit 209 for controlling the operation of the medication dispenser 201. The control unit 209 comprises a processor 210 that is programmed to carry out the functions that are needed to operate the medication dispenser 201, and a memory 211 for storing, for example, the medical regimen of the patient. The medical regimen is received from a server 212 over a communications network 213.

The taking time of the medication can be obtained from the medical regimen of the patient stored on the memory 211 of the control unit 209 or read from the label of the medication sachet 202. The medication dispenser 201 can be configured to dispense the medication automatically at the taking time or the dispensation of the medication may require a certain action from the patient, such as using a button 214 or a touch screen 215 of the medication dispenser 201.

The system of fig. 2 comprises a digital camera 216 for recording a digital video of the patient. The digital camera 216 is attached to the medication dispenser 201 with an adjustable support 217 that enables to adjust the digital camera 216 towards the patient. The digital camera 216 comprises a wide-angle lens (not shown in fig. 2). The digital camera 216 is connected to the control unit 209 of the medication dispenser 201. The control unit 209 is configured to control the operation of the digital camera 216. The digital video is stored into the memory 211 or sent directly to the server 212.

The recording of the digital video can be started at the time the medication is dispensed to the patient or at a predetermined time before or after the dispensation of the medication. Alternatively, the recording of the digital video can be started at the time the patient takes the medication from the medication dispenser 201 or at a predetermined time after the patient has taken the medication from the medication dispenser 201. The medication dispenser 201 comprises a sensor 218 for detecting the taking of the medication from the medication dispenser 201. Alternatively, the recording of the digital video can be started when the motion of the patient is detected. The digital camera 216 is used for detecting the motion of the patient. The recording of the digital video can be continued for a predetermined time or until the motion of the patient is not detected or has not been detected for a predetermined time. Alternatively, the recording of the digital video can be continued until the taking of the medication has been verified.

From the medication dispenser 201 the digital video is transmitted to the server 212 over the communications network 213. At the server 212, the digital video is stored in a database 219. The digital video is analysed at the server 212 to determine if the patient has taken the medication. The server 212 comprises a processor 220, a memory 221, and a computer program stored in the memory 221 that, when executed by the processor 220, analyses the digital video. The digital video can be analysed with a neural network, a frame-by-frame video motion analysis or a digital signal processing analysis technique. The determination if the patient has taken the medication can be based on the movement of a body, the opening of a mouth, or the movement of swallowing. If the taking of the medication has been verified, the time when the patient has taken the medication is determined and stored in the database 219. If the taking of the medication has not been verified, an alarm is given to the patient and/or the caregiver.

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A method for monitoring the taking of a medication, comprising:
- using a medication dispenser (201) to dispense the medication to a patient, **characterised in that** the method comprises:
- using a digital camera (216) to record a digital video of the patient, and
- analysing the digital video to determine if the patient has taken the medication.

2. The method according to claim 1, **characterised in that** the digital video is transmitted to a server (212) over a communications network (213), and the digital video is analysed at the server (212).

3. The method according to claim 1, **characterised in that** the digital video is analysed at the medication dispenser (201).

4. The method according to any of the preceding claims, **characterised in that** the step of using a digital camera (216) to record a digital video of the patient comprises adjusting continuously or at predetermined time intervals the orientation of the digital camera (216) so that the digital camera (216) is directed towards the patient's body.

5. The method according to any of the preceding claims, **characterised in that** the digital video is recorded with a plurality of digital cameras (216) arranged to capture the patient from different angles.

6. The method according to any of the preceding claims, **characterised in that** the recording of the digital video is started at the time the medication is dispensed to the patient.

7. The method according to any of claims 1 to 5, **characterised in that** the recording of the digital video is started at the time the patient takes the medication from the medication dispenser (201).

8. The method according to any of claims 1 to 5, **characterised in that** the recording of the digital video is started when the motion of the patient is detected.

9. The method according to any of the preceding claims, **characterised in that** the recording of the digital video is continued until the motion of the patient is not detected or has not been detected for a predetermined time.

10. The method according to any of claims 1 to 8, **characterised in that** the recording of the digital video is continued until the taking of the medication has been verified.

11. The method according to any of the preceding claims, **characterised in that** the determination if the patient has taken the medication is based on at least one of the following: movement of a body, opening of a mouth, and movement of swallowing.

12. The method according to any of the preceding claims, **characterised in that** the digital video is analysed using one of the following techniques: a neural network, a frame-by-frame video motion analysis or a digital signal processing analysis.

13. A medication system, comprising:
- a medication dispenser (201) configured to dispense a medication to a patient,
**characterised in that** the medication system comprises:
- a digital camera (216) configured to record a digital video of the patient, and
- means (210, 211, 220, 221) for analysing the digital video to determine if the patient has taken the medication.

14. The medication system according claim 13, **characterised in that** the medication system comprises means for transmitting the digital video to a server (212) over a communications network (213), and the means (220, 221) for analysing the digital video to determine if the patient has taken the medication are located at the server (212).

15. The medication system according to claim 13, **characterised in that** the means (210, 211) for analysing the digital video to determine if the patient has taken the medication are located at the medication dispenser (201).
